# EUROPÄISCHE PATENTSCHRIFT

(11) **EP 0 491 015 B1**
(45) Veröffentlichungstag und Bekanntmachung des Hinweises auf die Patenterteilung: **31.08.1994**
(21) Anmeldenummer: 91911143.5
(22) Anmeldetag: 21.06.1991
(51) Int. Cl.: B01D 65/02

(54) **VERFAHREN ZUM SPÜLEN DER FILTRATIONS-MODULE EINER ANLAGE ZUR KLÄRUNG VON FLÜSSIGKEITEN**
PROCESS FOR RINSING THE FILTER MODULE OF A DEVICE FOR PURIFYING LIQUIDS
PROCEDE POUR RINCER DES MODULES DE FILTRATION D'UNE INSTALLATION D'EPURATION DE LIQUIDES

(30) Priorität: 06.07.1990 CH 2270/90
(43) Veröffentlichungstag der Anmeldung: 24.06.1992
(73) Patentinhaber: BUCHER-GUYER AG Maschinenfabrik, CH-8166 Niederweningen (CH)
(72) Erfinder: HARTMANN, Eduard, CH-5425 Schneisingen (CH)
(86) Internationale Anmeldenummer: CH9100134
(87) Internationale Veröffentlichungsnummer: WO9200797

(56) Entgegenhaltungen:
- EP-A- 0 321 751
- WO-A-88/00494
- DE-A- 3 818 437
- Chemie-Ingenieur-Technik, Vol. 61, No. 6, June 1989, VCH Verlagsgesellschaft mbH, (Weinheim, DE) T. Faust et al.: "Einsatz von Cross-flow-Techniken in der Bioverfahrenstechnik", pages 459-468

## Beschreibung

Die Erfindung betrifft ein Verfahren zum Spülen der Filtrations-Module einer Anlage zur Klärung von Flüssigkeiten, insbesondere Rohsaft aus pflanzlichen Produkten oder biotechnologisch hergestellten Produkten mittels Querstromfiltration, insbesondere Mikro- oder Ultrafiltration, die mit einem hohen Feststoffanteil im Retentat-Umwälzkreislauf gefahren wird.

Bei dem aus pflanzlichen Produkten gewonnenen Rohsaft kann es sich sowohl um alkoholische als auch um nichtalkoholische Rohsäfte handeln, wie z.B. Rohsaft aus Obst, Trauben, Beeren oder anderen Früchten und Gemüsen sowie Oelsaaten zur Oelgewinnung. Es kommen auch daraus abgeleitete Produkte aus mehreren pflanzlichen Produkten, z.B. Bier, insbesondere im Zusammenhang mit der Bier-Rückgewinnung aus Fermenter- und Tanklager-Hefe in Betracht.

Durch die WO 89/02708 ist es bekannt, bei der Klärung von Flüssigkeiten mit einem hohen Feststoffanteil im Retentat-Umwälzkreislauf zu fahren. Damit wird das Ziel verfolgt, die Filtrationsleistung der Membranen massgebend zu verbessern.

Aufgrund des hohen Feststoffanteils im Retentat-Umwälzkreislauf besteht bei derartigen Anlagen die Gefahr, dass die Filtrations-Module der Querstromfiltrationseinrichtung verstopfen. Vor allem beim plötzlichen Abschalten der Anlage, wie z.B. bei Stromausfall, Ausfall von Steuerluft für die pneumatisch betriebenen Einrichtungen etc., müssen sämtliche Retentat-Kanäle des Querstromfiltrationssystems raschmöglichst freigespült werden. Wenn dies nicht sofort geschieht, nimmt die Viskosität des Retentats zu, weil das Retentat eine tyxotropische Flüssigkeit ist und diese beim Stillstand der Retentat-Umwälzpumpe nicht mehr bewegt wird. Dies hat zur Folge, dass die Filtrations-Module unter Umständen nicht mehr mit Spülmittel freigespült werden können respektive mit grossem Aufwand von Kanal zu Kanal mechanisch, meistens handwerklich, gereinigt werden müssen. Im allgemeinen ist dies nur bei grobkanaligen Modulen möglich, feinkanalige Module verstopfen irreversibel.

Dieses Problem wird noch dadurch verstärkt, dass oftmals mit einem überdurchschnittlich hohen Trubanteil im Retentat-Kreislauf gefahren werden muss, um die Querstromfiltration mit drastisch erhöhter Filtrationsleistung zu betreiben.

Um diese Nachteile zu vermeiden ist es bekannt, wenn die Anlage abstellt, dem Retentat-Kreislauf Wasser zuzuführen, um die Module zu spülen. Die Wasserzufuhr erfolgt stromaufwärts zur Kreislauf-Pumpe für das Retentat und wird in bekannter Weise mit Hilfe von Auf/Zu-Armaturen durchgeführt. Mit der Zufuhr von Wasser wird das Retentat verdünnt, so dass die Viskosität sinkt und die Anlage anschliessend wieder normal gestartet werden kann.

Für die Retentatzufuhr zum Modul bzw. Modul-Pass, das sind mehrere in Serie geschaltete Module, ist es bekannt, die einzelnen Modul-Passe bzw. die Einzelmodule zwecks einfacher Rohrleitungsführung asymmetrisch anzuströmen. Hierzu zweigen von der Hauptleitung mehrere Nebenleitungen nacheinander zu den einzelnen Modul-Passe oder zu den Einzelmodulen ab. Das gleiche gilt auch für den Retentatabfluss ab Modul-Passe. Eine andere, bekannte Ausführung verwendet drei Module, die durch eine Verzweigung der Hauptleitung in drei einzelne Leitungen symmetrisch angeströmt werden.

Die Zugabe von Wasser als Spülmittel zum Retentatkreislauf und die bekannte Art der Zufuhr des Retentats zum Modul-Pass, insbesondere der symmetrische Zu- und Abfluss, können in einfachen Fällen dazu beitragen, die Verstopfungsgefahr der Module zu verringern. Wenn jedoch, wie im vorliegenden Fall, die Anlage mit einem sehr hohen Feststoffanteil im Retentat-Kreislauf gefahren wird, reichen diese bekannten Massnahmen in den genannten Notsituationen nicht mehr aus, um die Filtrationsmodule mit Wasser freizuspülen.

Der Erfindung liegt deshalb die Aufgabe zugrunde, ein Verfahren bzw. eine Sicherheitsvorkehrung für die eingangs erwähnte Anlage zu schaffen, die einen störungsfreien Arbeitsablauf durch Beseitigung der Verstopfungsgefahr bei den Filtrations-Modulen, insbesondere bei einem sehr hohen Feststoffanteil im Retentat-Kreislauf, gewährleistet.

Gemäss der Erfindung wird diese Aufgabe dadurch gelöst, dass die Retentatseite der Querstromfiltrations-Module beim Abstellen der Anlage mit zeitlich zunehmender und/oder geometrisch zunehmender Spülmittelzugabe beschickt wird.

Weitere Merkmale und vorteilhafte Ausgestaltungen der Erfindung sind den Patentansprüchen zu entnehmen.

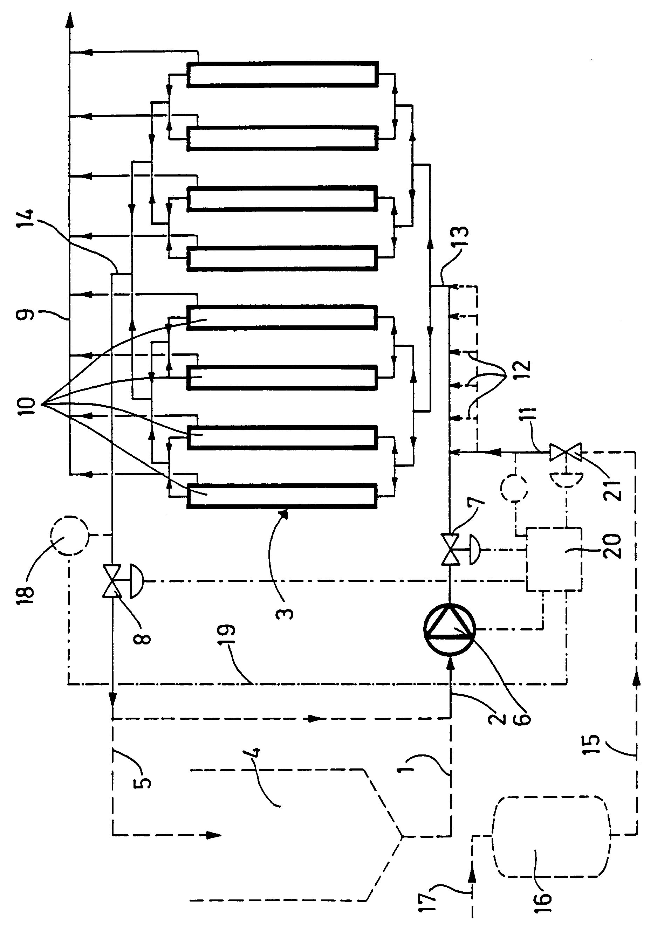

Die Erfindung ist in der folgenden Beschreibung und der Zeichnung, die ein Ausführungsbeispiel in schematischer Darstellung zeigt, näher erläutert.

Der zu klärende Rohsaft wird über eine Leitung 1 einem Retentat-Umwälzkreislauf 2 einer Querstromfiltrationseinrichtung 3 zugeführt, die vorzugsweise aus einer Mikro- oder Ultrafiltrationseinrichtung besteht. Die Anlage kann ein- oder mehrstufig kontinuierlich betrieben werden. Das erfindungsgemässe Verfahren kann jedoch auch für eine semikontinuierliche, im Batch-Verfahren betriebene Querstromfiltration angewendet werden. Hierzu ist die Leitung 1 an einen Puffer-Tank 4 angeschlossen, in den das Retentat über eine Leitung 5 zurückgeführt wird (in der Zeichnung mit gestrichelten Linien als Alternative dargestellt). Im Retentat-Umwälzkreislauf 2 der im Ausführungsbeispiel einstufigen und kontinuierlichen Querstromfiltrationseinrichtung 3 ist eine Umwälzpumpe 6, ein Stufen-Eingangsventil 7 und ein Stufen-Ausgangsventil 8 angeordnet. Das Permeat der Querstromfiltrationseinrichtung 3 wird durch eine Leitung 9 abgeführt. Zur Verbesserung der Filtrationsleistung wird die Anlage mit einem sehr hohen Feststoffanteil im Retentat-Umwälzkreislauf 2 betrieben. Der dadurch entstehenden Verstopfungsgefahr der Filtrations-Module 10 der Querstromfiltrationseinrichtung 3, insbesondere beim Abstellen der Anlage beispielsweise durch Stromausfall, wird durch Zuführung von Spülmittel, vorzugsweise Wasser, begegnet. Hierzu ist eine Leitung 11 vorgesehen, die auf der Retentatseite der Querstromfiltrationseinrichtung 3 in den Retentat-Umwälzkreislauf 2 nach der Umwälzpumpe 6 und dem Stufen-Eingangsventil 7 mündet.

Zur Erzielung einer bestmöglichen Spülwirkung wird das Spülmittel mit zeitlich zunehmender und/oder geometrisch zunehmender Zufuhr in den Retentat-Umwälzkreislauf 2 eingeleitet. Dabei kann die Spülmittel-Zufuhr stetig oder schrittweise erfolgen. Eine rein zeitlich zunehmende Spülmittelzugabe wird beispielsweise dadurch erreicht, dass mit einer kleinen Mengenleistung an Spülmittel begonnen und diese mit der Zeit vorzugsweise langsam gesteigert wird, statt von Anfang an mit einer bestimmten, konstanten Spülmittelmenge zu spülen. Die Spülmittel-Zugabe kann auch mit einem relativ viskosen Spülmittel beginnen, dessen Viskosität etwas geringer ist als das Retentat, wobei die Viskosität des Spülmittels mit der Zeit verringert wird. Dies kann bspw. durch Zudosieren von Wasser zum Spülmittel geschehen.

Eine rein geometrisch zunehmende Spülmittelzufuhr kann bspw. durch eine fraktionierte Zugabe des Spülmittels an mehreren Stellen des Retentat-Umwälzkreislaufes 3 erfolgen, wobei die totale Menge hier zeitlich konstant sein kann. Die fraktionierte Zugabe wird im Ausführungsbeispiel durch die Verzweigung der Leitung 11 in mehrere Einzelleitungen 12 erreicht, die nach dem Stufen-Eingangsventil 7 nacheinander in den Retentat-Umwälzkreislauf 3 münden.

Die Dauer der zunächst kleinen Spülmittelleistung sollte bis zur vollen Spülleistung wenigstens 30 Sekunden betragen. Die volle Spülleistung ist normalerweise dadurch gegeben, dass nach der Wasserzugabe wieder der normale Leitungsdruck an der Zugabestelle in der Retentat-Leitung herrscht.

Aufgrund der genannten, relativ sanften Massnahmen wird erreicht, dass sich das zähere Retentat mit dem Spülmittel vermischt, so dass ein Freispülen des Retentates in den Filtrations-Modulen 10 möglich wird. Es kommt nicht zu einer Kanalbildung von relativ dünnviskosem Spülmittel (Wasser) durch das viskose Retentat, die eine vollständige Spülung verhindert.

Die Zufuhr von Spülmittel kann im Prinzip an jeder Stelle im Retentat-Umwälzkreislauf 2, vorzugsweise jedoch stromabwärts nach der Umwälzpumpe 6 erfolgen, damit eine ausreichende Sicherheit gegeben ist, wenn die Umwälzpumpe 6 wegen Stromausfall nicht mehr läuft.

Als Spülmittel wird normalerweise Wasser verwendet. In besonderen Fällen können jedoch auch viskosere Spülmittel, z.B. Retentat, vorzugsweise geschwefelt, aus vorhergehenden Filtrationszyklen oder Austrag aus kontinuierlicher Filtration eingesetzt werden. In unkritischen Fällen ist auch eine für diesen Zweck hergestellte Suspension aus artfremden Stoffen möglich.

Die Filtrations-Module 10 der Querstromfiltrationseinrichtung 3 sind vorzugsweise grobkanalig mit einem Kanal-Durchmesser bzw. einer Kanal-Weite von wenigstens 4 mm ausgeführt. Vorzugsweise werden Rohrmodule verwendet, weil hiermit eine besonders gleichmässige Strömung erreicht wird und keine Ecken mit strömungstoten Stellen vorhanden sind.

Im Ausführungsbeispiel sind acht Einzeln-Filtrations-Module oder acht Modul-Presse mit mehreren in Serie geschalteten Einzelmodulen angeordnet vorgesehen. Die Retentatzufuhr zu den Filtrations-Modulen 10 erfolgt symmetrisch über eine Leitung 13, die sich in zwei Leitungen verzweigt, von denen sich wiederum jede Leitung in zwei weitere Leitungen verzweigt, so dass die Filtrations-Module 10 symmetrisch und hierarchisch zentral angeströmt werden. Die gleiche Leitungsverzweigung erfolgt auf der Abflussseite der Filtrations-Module 10 durch Zusammenfassen der Retentatabflussleitungen zu einer Leitung 14, die im Retentat-Umwälzkreislauf 2 liegt.

In weiteren Ausführungsbeispielen kann jede Anlagestufe der Querstromfiltrationseinrichtung 3 vorzugsweise aus zwei, vier, acht, sechszehn etc. Modul-Passe bzw. Einzelmodulen bestehen. Die Anströmung bzw. der Abfluss kann hierarchisch zentral in Gruppen von zwei, vier, acht, sechzehn etc. Modul-Passe bzw. Einzelmodulen erfolgen.

Durch die genannte Anordnung und Anströmung der Filtrations-Module 10 ist eine gleichmässige Durchspülung aller Filtrations-Module gewährleistet. Es wird somit vermieden, dass z.B. einzelne Filtrations-Module weniger gut gespült werden und dadurch die Verstopfungsgefahr vergrössert wird.

Vorzugsweise wird die Querstromfiltrationseinrichtung 3 über eine Leitung 15 mit Spülmittel aus einem Speicher 16 versorgt, der bspw. enthärtetes Wasser oder Brüdenwasser enthält und über eine Leitung 17 an eine Druckluftquelle angeschlossen ist. Damit ist das Spülmittel in Notfällen sehr rasch verfügbar.

Zur Automatisierung der zeitlich allmählich verstärkenden Spülmittel-Zugabe wird die Zugabemenge und/oder die Viskositätsabnahme des Spülmittels gesteuert oder geregelt. Die Steuerung kann nach einem Zeitprogramm in Schritten oder stetig verändernd erfolgen. Die Regelung kann in Abhängigkeit einer durch die Spülung beeinflussten Prozess-Variablen durchgeführt werden. Beide Massnahmen zur Steuerung und Regelung können auch kombiniert eingesetzt werden. Im Ausführungsbeispiel wird die Durchflussmenge des Retentats nach den Filtrations-Modulen 10 durch eine im Retentat-Umwälzkreislauf 2 angeordnete Messstelle 18 gemessen und über eine Steuerleitung 19 an ein Steuer-/Regelorgan 20 übermittelt. Von dort aus wird eine in der Spülmittel-Zufuhr-Leitung 11 angeordnetes Regelventil 21 angesteuert.

Unabhängig von diesen Steuer- bzw. Regelvorgängen wird z.B. bei Stromausfall das Stufen-Eingangsventil 7 automatisch geschlossen und das Stufen-Ausgangsventil 8 voll geöffnet.

Aufgrund dieser Massnahmen ist es auch möglich, eine mit sehr hohem Trubanteil im Retentatkreislauf betriebene Querstromfiltrations-Anlage durch Knopfdruck bzw. von aussen angesteuert automatisch abstellen zu lassen, ohne dass Verstopfungsgefahr für die Filtrations-Module besteht. Neben der einfacheren Bedienung entstehen dadurch weitere Automatisierungsmöglichkeiten im Zusammenhang mit der Automatisierung ganzer Produktionslinien.

Die Anwendung der vorliegenden Erfindung ist nicht beschränkt auf die beschriebenen Anwendungsfalle. Sie kann auch mit Vorteil eingesetzt werden, wenn aus wirtschaftlichen Gründen, gegeben durch die Produktverluste, möglichst hohe Ausbeuten erzielt werden sollen (teure Produkte).

Eine weitere Anwendung ist möglich, wenn aus wirtschaftlichen Gründen, gegeben durch die Gesamtprozesskosten z.B. bei nachfolgender Trocknung oder Deponie, ein möglichst trockenes Produkt erzielt werden soll, wie dies bspw. in der Abwassertechnik der Fall ist.

Ferner ist die Anwendung der Erfindung nicht auf das beschriebene Modul-System beschränkt. Sie lässt sich vielmehr im Prinzip für alle bekannten Modul-Systeme mit Vorteil einsetzen.

## Patentansprüche

1. Verfahren zum Spülen der Filtrations-Module einer Anlage zur Klärung von Flüssigkeiten, insbesondere Rohsaft aus pflanzlichen Produkten oder biotechnologisch hergestellten Produkten, mittels Querstromfiltration, insbesondere Mikro- oder Ultrafiltration, die mit einem hohen Feststoffanteil im Retentat-Umwälzkreislauf gefahren wird, dadurch gekennzeichnet, dass die Retentatseite der Filtrations-Module beim Abstellen der Anlage mit zeitlich und/oder geometrisch zunehmender Spülmittelzugabe beschickt wird.

2. Verfahren nach Anspruch 1, dadurch gekennzeichnet, dass die Spülmittelzugabe stetig oder schrittweise erfolgt.

3. Verfahren nach Anspruch 1 oder 2, dadurch gekennzeichnet, dass die Spülmittelzugabe mit kleiner Mengenleistung beginnt und mit der Zeit vorzugsweise langsam gesteigert wird und/oder dass die Spülmittelzugabe mit einem relativ viskosen Spülmittel beginnt, dessen Viskosität vorzugsweise etwas geringer ist als das Retentat, und dass die Viskosität des Spülmittels mit der Zeit verringert wird.

4. Verfahren nach Anspruch 3, dadurch gekennzeichnet, dass die Dauer der Spülmittelzugabe bis zur vollen Spülleistung wenigstens 30 Sekunden beträgt.

5. Verfahren nach Anspruch 4, dadurch gekennzeichnet, dass die Viskosität des Spülmittels durch Zudosieren von Wasser verringert wird.

6. Verfahren nach einem der Ansprüche 1 bis 5, dadurch gekennzeichnet, dass eine fraktionierte Zugabe des Spülmittels an mehreren Stellen des Retentat-Umwälzkreislaufes erfolgt, wobei die totale Spülmittelmenge zeitlich konstant sein kann.

7. Verfahren nach einem der Ansprüche 1 bis 6, dadurch gekennzeichnet, dass die Zufuhr von Spülmittel an jeder beliebigen Stelle des Retentat-Umwälzkreislaufes, jedoch vorzugsweise stromabwärts zur Umwälzpumpe erfolgt.

8. Verfahren nach einem der Ansprüche 1 bis 7, dadurch gekennzeichnet, dass das Spülmittel eine höhere Viskosität als Wasser besitzt.

9. Verfahren nach Anspruch 8, dadurch gekennzeichnet, dass das Spülmittel aus Retentat einer vorhergehenden Filtration besteht, welches vorzugsweise geschwefelt ist.

10. Verfahren nach Anspruch 8, dadurch gekennzeichnet, dass das Spülmittel aus einer Suspension von artfremden Stoffen besteht.

11. Verfahren nach einem der Ansprüche 1 bis 10, dadurch gekennzeichnet, dass die zu spülende Querstromfiltrationseinrichtung mit grobkanaligen Modulen mit einem Kanaldurchmesser bzw. einer Kanalweite von wenigstens 4 mm bestückt ist.

12. Verfahren nach Anspruch 11, dadurch gekennzeichnet, dass Rohrmodule verwendet werden.

13. Verfahren nach einem der Ansprüche 1 bis 12, dadurch gekennzeichnet, dass das Spülmittel einem Speicher für enthärtetes Wasser oder Brüdenwasser entnommen wird.

14. Verfahren nach einem der Ansprüche 1 bis 13, dadurch gekennzeichnet, dass die Menge der Spülmittelzugabe und/oder die Viskositätsabnahme des Spülmittels gesteuert und/oder geregelt wird.

15. Verfahren nach Anspruch 14, dadurch gekennzeichnet, dass die Steuerung durch ein Zeitprogramm schrittweise oder stetig verändernd erfolgt.

16. Verfahren nach Anspruch 14 oder 15, dadurch gekennzeichnet, dass die Regelung in Abhängigkeit einer durch die Spülung beeinflussten Prozess-Variablen, bspw. der Durchflussmenge nach den Modulen der Querstromfiltration erfolgt.

17. Verfahren nach einem der Ansprüche 1 bis 16, dadurch gekennzeichnet, dass die Modul-Passe bzw. Einzelmodule symmetrisch angeströmt werden.

18. Verfahren nach einem der Ansprüche 1 bis 17, dadurch gekennzeichnet, dass der Abfluss von den Modul-Passen oder den Einzelmodulen symmetrisch erfolgt.

19. Verfahren nach einem der Ansprüche 1 bis 18, dadurch gekennzeichnet, dass eine Anlage-Stufe aus zwei, vier, acht, sechzehn, etc. Modul-Passe bzw. Einzelmodulen besteht.

20. Verfahren nach einem der Ansprüche 1 bis 19, dadurch gekennzeichnet, dass die Anströmung der Filtrations-Module hierarchisch zentral in Gruppen von zwei, vier, acht, sechzehn etc. Modulen erfolgt.

21. Verfahren nach einem der Ansprüche 1 bis 20, dadurch gekennzeichnet, dass der Abfluss von den Filtrations-Modulen hierarchisch zentral in Gruppen von zwei, vier, acht, sechzehn etc. Modulen erfolgt.

22. Verfahren nach einem der Ansprüche 1 bis 21, dadurch gekennzeichnet, dass der Betrieb der Querstromfiltrations-Anlage durch Knopfdruck bzw. von aussen angesteuert automatisch abstellbar ist.

## Claims

1. Process for rinsing the filter modules of a device for purifying liquids, especially raw juice from vegetable products or biotechnological products, by means of cross-flow filtration, especially micro- or ultrafiltration, run with a high solids content in the residue circuit, characterised in that the residue side of the filter modules is supplied with a quantity of rinsing agent which increases in time and/or geometrically when the device stops.

2. Process according to claim 1, characterised in that the rinsing agent is added continuously or stepwise.

3. Process according to claim 1 or claim 2, characterised in that the rinsing agent is initially added in a low volume which is preferably increased slowly in time and/or that the rinsing agent is initially added in the form of a relatively viscous rinsing agent, the viscosity of which is preferably slightly lower than that of the residue, and that the viscosity of the rinsing agent is reduced in time.

4. Process according to claim 3, characterized in that the duration of the addition of rinsing agent up to full rinsing efficiency is at least 30 seconds.

5. Process according to claim 4, characterised in that the viscosity of the rinsing agent is reduced by the metered addition of water.

6. Process according to one of claims 1 to 5, characterised in that the rinsing agent is added in fractions at several points of the residue circuit, wherein the total quantity of rinsing agent can be constant in time.

7. Process according to one of claims 1 to 6, characterised in that the rinsing agent is supplied to any desired point of the residue circuit, although preferably downstream of the circulating pump.

8. Process according to one of claims 1 to 7, characterised in that the rinsing agent has a higher viscosity than water.

9. Process according to claim 8, characterized in that the rinsing agent consists of residue from a preceding filtration process, this residue preferably being sulphurised.

10. Process according to claim 8, characterised in that the rinsing agent consists of a suspension of substances of different compositions.

11. Process according to one of claims 1 to 10, characterised in that the cross-flow filtration device to be rinsed is provided with large-channel modules with a channel diameter or a channel width of at least 4 mm.

12. Process according to claim 11, characterised in that tubular modules are used.

13. Process according to one of claims 1 to 12, characterised in that the rinsing agent is taken from a reservoir for softened water or condenser water.

14. Process according to one of claims 1 to 13, characterised in that the quantity of rinsing agent added and/or the reduction in the viscosity of the rinsing agent is controlled.

15. Process according to claim 14, characterised in that control is effected in a stepwise or continuously varying manner by a time programme.

16. Process according to claim 14 or claim 15, characterised in that control is effected as a function of a process variable influenced by the rinsing process or of the flow rate downstream of the modules of the cross-flow filtration.

17. Process according to one of claims 1 to 16, characterised in that the module passes or individual modules are traversed symmetrically.

18. Process according to one of claims 1 to 17, characterised in that the outflow from the module passes or individual modules is effected symmetrically.

19. Process according to one of claims 1 to 18, characterized in that a device stage consists of two, four, eight, sixteen, etc., module passes or individual modules.

20. Process according to one of claims 1 to 19, characterised in that the filter modules are traversed hierarchically centrally in groups of two, four, eight, sixteen, etc. modules.

21. Process according to one of claims 1 to 20, characterised in that the outflow from the filter modules is effected hierarchically centrally in groups of two, four, eight, sixteen, etc. modules.

22. Process according to one of claims 1 to 21, characterised in that the operation of the cross-flow filtration device can be stopped automatically by the push of a button or by external control means.

## Revendications

1. Procédé pour rincer les modules de filtration d'une installation d'épuration ou de clarification de liquides, en particulier de jus brut de produits végétaux ou de produits élaborés par voie biotechnologique, au moyen d'une filtration à courant transversal, en particulier d'une micro- ou ultrafiltration, installation que l'on fait fonctionner avec une haute teneur de matières solides dans le circuit de recyclage de rétentat, caractérisé en ce que, lors d'un arrêt de l'installation, on applique au côté rétentat des modules de filtration une addition d'agent de rinçage augmentant dans le temps et/ou géométriquement.

2. Procédé selon la revendication 1, caractérisé en ce que l'on effectue l'addition d'agent de rinçage de façon continue ou pas à pas.

3. Procédé selon la revendication 1 ou 2, caractérisé en se que l'on commence l'addition d'agent de rinçage à un faible débit, que l'on augmente avec le temps, de préférence lentement, et/ou que l'on commence l'addition d'agent de rinçage par un agent de rinçage relativement visqueux dont la viscosité est de préférence légèrement inférieure à celle du rétentat, et que l'on réduit la viscosité de l'agent de rinçage avec le temps.

4. Procédé selon la revendication 3, caractérisé en ce que la durée de l'addition d'agent de rinçage jusqu'à l'obtention de la pleine capacité de rinçage est d'au moins 30 secondes.

5. Procédé selon la revendication 4, caractérisé en ce que l'on réduit la viscosité de l'agent de rinçage par addition dosée d'eau.

6. Procédé selon une des revendications 1 à 5, caractérisé en ce que l'on effectue une addition fractionnée de l'agent de rinçage à plusieurs endroits du circuit de recyclage de rétentat, le débit total d'agent de rinçage pouvant être constant dans le temps.

7. Procédé selon une des revendications 1 à 6, caractérisé en ce que l'amenée d'agent de rinçage peut s'effectuer à n'importe quel endroit désiré du circuit de recyclage de rétentat, mais s'effectue de préférence en aval de la pompe de circulation.

8. Procédé selon une des revendications 1 à 7, caractérisé en ce que l'agent de rinçage possède une plus grande viscosité que l'eau.

9. Procédé selon la revendication 8, caractérisé en ce que l'agent de rinçage est du rétentat provenant d'une filtration précédente et auquel on a de préférence fait subir une sulfuration.

10. Procédé selon la revendication 8, caractérisé en ce que l'agent de rinçage est une suspension de matières dont la nature diffère de celle des liquides traités.

11. Procédé selon une des revendications 1 à 10, caractérisé en ce que le dispositif de filtration à courant transversal qu'il s'agit de rincer, est muni de modules à gros canaux, ayant un diamètre ou une largeur de canal d'au moins 4 mm.

12. Procédé selon la revendication 11, caractérisé en ce que le dispositif est muni de modules tubulaires.

13. Procédé selon une des revendications 1 à 12, caractérisé en ce que l'on prélève l'agent de rinçage d'un réservoir pour de l'eau ayant subi un traitement d'adoucissement ou pour de l'eau de condensation.

14. Procédé selon une des revendications 1 à 13, caractérisé en ce que l'on commande et/ou règle le débit de l'agent de rinçage ajouté et/ou la réduction de la viscosité de l'agent de rinçage.

15. Procédé selon la revendication 14, caractérisé en ce que la commande s'effectue avec une variation pas à pas ou continue par un programme en fonction du temps.

16. Procédé selon la revendication 14 ou 15, caractérisé en ce que la régulation s'effectue en fonction d'une variable du processus influencée par le rinçage, par exemple en fonction du débit en aval des modules du dispositif de filtration à courant transversal.

17. Procédé selon une des revendications 1 à 16, caractérisé en ce que l'alimentation des passes de modules ou des modules individuels est symétrique.

18. Procédé selon une des revendications 1 à 17, caractérisé en ce que l'évacuation des passes de modules ou des modules individuels est symétrique.

19. Procédé selon une des revendications 1 à 18, caractérisé en ce qu'un étage de l'installation se compose de deux, quatre, huit, seize, et ainsi de suite, passes de modules ou modules individuels.

20. Procédé selon une des revendications 1 à 19, caractérisé en ce que l'alimentation des modules de filtration s'effectue de façon hiérarchiquement centralisée en groupes de deux, quatre, huit, seize, et ainsi de suite, modules.

21. Procédé selon une des revendications 1 à 20, caractérisé en ce que l'évacuation des modules de filtration s'effectue de façon hiérarchiquement centralisée en groupes de deux, quatre, huit, seize, et ainsi de suite, modules.

22. Procédé selon une des revendications 1 à 21, caractérisé en ce que le fonctionnement de l'installation de filtration à courant transversal peut être arrêté en appuyant sur un bouton ou de façon automatique par une commande extérieure.
